# EUROPÄISCHE PATENTSCHRIFT

(11) **EP 1 062 262 B1**
(45) Veröffentlichungstag und Bekanntmachung des Hinweises auf die Patenterteilung: **01.10.2003**
(21) Anmeldenummer: 99910312.0
(22) Anmeldetag: 01.03.1999
(51) Int. Cl.: C08G 64/30, C07F 9/54, B01J 27/20

(54) **VERFAHREN ZUR HERSTELLUNG EINER FLÜSSIGEN FORMULIERUNG VON TETRAPHENYLPHOSPHONIUMPHENOLAT**
METHOD FOR PRODUCING A LIQUID FORMULATION OF TETRAPHENYLPHOSPHONIUM PHENOLATE
PROCEDE POUR PRODUIRE UNE FORMULATION LIQUIDE DE PHENOLATE DE TETRAPHENYLPHOSPHONIUM

(30) Priorität: 12.03.1998 DE 19810745
(43) Veröffentlichungstag der Anmeldung: 27.12.2000
(73) Patentinhaber: BAYER AG, 51368 Leverkusen (DE)
(72) Erfinder: KÖNIG, Annett, D-47800 Krefeld (DE); PREIN, Michael, D-47802 Krefeld (DE)
(86) Internationale Anmeldenummer: EP9901330
(87) Internationale Veröffentlichungsnummer: WO99046315

(56) Entgegenhaltungen:
- EP-A- 0 798 329
- EP-A- 0 826 693
- WO-A-99/00395
- US-A- 3 442 854
- US-A- 4 302 574

## Beschreibung

Die vorliegende Erfindung betrifft eine bei Raumtemperatur flüssige Katalysatorformulierung aus Tetraphenylphosphoniumphenolat und Phenol und deren Verwendung als Umesterungskatalysator, insbesondere zur lösungsmittelfreien Herstellung von thermoplastischen Polycarbonaten.

Die Herstellung von Phosphoniumphenolaten ist aus DE-OS 196 35 656 und der deutschen Patentanmeldung P 19727351.3 bekannt. Aus der US-PS 3.442.854 ist u.a. bekannt, daß sich Phosphoniumphenolate, gegebenenfalls unter Mitverwendung von Alkali-/Erdalkaliverbindungen, besonders als Katalysatoren zur Veresterung und zur Umesterung, insbesondere zur Herstellung von Polycarbonaten nach dem Schmelzumesterungsverfahren eignen. Da die Katalysatordosierung als Feststoff technisch Schwierigkeiten mit sich bringt, ist es wünschenswert, den Katalysator flüssig zu dosieren. Beispielsweise kann der Katalysator in flüssigem Phenol, d.h. bei Temperaturen oberhalb 45°C, im allgemeinen bei ca. 60°C, gelöst werden und als Lösung dem Verfahren zugeführt werden. Dies erfordert eine temperierte Vorlage und damit zusätzlichen technischen Aufwand. Aus J.Org. Chem. 32 (1967) 1060 ist zudem bekannt ist, daß Phosphoniumphenolate thermolabil sind, so daß eine thermische Vorschädigung insbesondere durch langes Verweilen der Katalysatorlösung in der temperierten Vorlage nicht auszuschließen ist. Eine andere Möglichkeit bietet eine Dosierung aus einer bei Raumtemperatur flüssigen Mischung aus Phenol und Wasser (90 Gew.-% : 10 Gew.-%). Nachteilig hierbei ist, daß Wasser als zusätzlicher Stoff ins Verfahren eingebracht wird. Außerdem wird in Liebigs Ann. Chem. Bd. 634 (1960) 1 die alkalische Zersetzung von quartären Phosphoniumsalzen zu Triphenylphosphinoxid beschrieben. Eigenen Untersuchungen zufolge bewirken auch geringe Mengen Wasser bei hohen Temperaturen eine Spaltung von Tetraphenylphosphoniumphenolat (TPP-P) zu Triphenylphosphanoxid.

Aufgabe der vorliegenden Erfindung war es daher, eine einfache Möglichkeit zur Flüssig-Dosierung von Tetraphenylphosphoniumphenolat bereitzustellen. Es wurde nun gefunden, daß eine Mischung von TPP-P, bzw. des Addukts von TPP-P mit 2 Molekülen Phenol, im folgenden mit TPP-P*2PhOH bezeichnet, mit Phenol bei einer Zusammensetzung von 28 bis 45 Gew.-% TPP-P (bzw. 40-65 Gew.-% TPP-P*2PhOH) - und entsprechend 72 bis 55 Gew.-% (bzw. 60 bis 35 Gew.-%) Phenol bei Raumtemperatur flüssig ist. Dies ermöglicht die Katalysatorzugabe im flüssigen Zustand ohne Fremd- oder Zusatzstoffe sowie ohne thermische Vorbelastung des Katalysators.

Die Verwendung der erfindungsgemäß erhaltenen flüssigen Formulierung von Tetraphenylphosphoniumphenolat als Katalysator zur Herstellung von aromatischen Polycarbonaten kann in an sich bekannter Weise erfolgen (s. beispielsweise US-PS 3,442,854). Gemäß dem dort offenbarten Schmelzumesterungsverfahren werden beispielsweise aus aromatischen Diphenolen, Kohlensäurediarylestem und gegebenenfalls Verzweigem und/oder Monophenolen aromatische Polycarbonate hergestellt.

Die erfindungsgemäß hergestellten Phosphoniumphenolate können als Umesterungs-Katalysatoren in Mengen von 10⁻¹ mol bis 10⁻⁸ mol, vorzugsweise in Mengen von 10⁻³ mol bis 10⁻⁷ mol, pro mol Diphenol eingesetzt werden.

Weitere Einzelheiten des Schmelzumesterungsverfahrens sind in der Literatur beschrieben (siehe beispielsweise Hermann Schnell, Chemistry and Physics of Polycarbonates, Polymer Reviews, Vol. 9, 1964, Seiten 44 bis 51, DE-AS 1 031 512, US-PS 3 022 272, US-PS 5 340 905 und US-PS 5 399 659).

Die mit der erfindungsgemäßen flüssigen Formulierung von TPP-P hergestellten thermoplastischen Polycarbonate sind lösungsmittelfrei, mit heller Eigenfarbe ausgestattet und weitgehend frei von unerwünschten Fehlstellen im Polycarbonat. Weitgehend frei von unerwünschten Fehlstellen im Polycarbonat im Sinne des erfindungsgemäßen Verfahrens heißt, daß der Gehalt an Verzweiger der Formel (I) 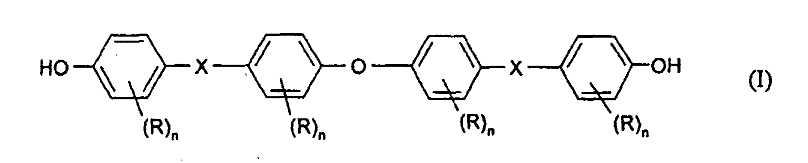 mit
- X=: C₁-C₉,-Alkyliden oder Cycloalkyliden, S oder eine Einfachbindung und
- (R)ₙ=: jeweils unabhängig voneinander COOH, CH₃, Cl oder Br, wobei an einen Phenylring auch mehrere unterschiedliche (R)ₙ auftreten können
- n =: 0,1,2 oder 3,
im Polycarbonat einen Wert nach Totalverseifung und HPLC-Bestirnmung von 300 ppm nicht übersteigt.

### Beispiele

### Herstellung von verschiedenen Phenol/ TPP-P*2PhOH-Mischungen

Phenol und TPP-P*2PhOH wurden entsprechend den Zusammensetzungen in Tabelle 1 unter N₂-Atmosphäre in 20 ml Rollrandfläschchen mit Magnetrührer eingewogen (Gesamtmenge jeweils 10 g) und mit einem Septum verschlossen. Diese Fläschchen wurden in eine automatische Schütteleinrichtung gespannt und in ein temperierbares Wasserbad getaucht. Die Temperatur des Wasserbades wird nun schrittweise (5°C/h) solange erhöht, bis die Mischungen flüssig vorliegen, gleichzeitig sorgt die Schütteleinrichtung für eine optimale Vermischung. Nach dem Aufschmelzen werden die Proben aus dem Wasserbad genommen und auf Raumtemperatur abgekühlt Die Proben 4a, 5, 6 und 7 blieben flüssig, die anderen bildeten eine feste und flüssige Phase bzw. wurden fest In der Tabelle 1 sind von allen Proben die Schmelztemperaturen (Tₘₑₗₜ) zusammengefaßt. Diese wurden bestimmt, indem die aufgeschmolzenen und auf Raumtemperatur abgekühlten Proben mittels Wasserbad solange erwärmt wurden (5°C/h), bis sie wieder flüssig waren.

**Tabelle 1**

| Zusammensetzung der Katalysatorformulierungen und deren Schmelzpunkte | | | | | |
|---|---|---|---|---|---|
| **Probe-Nr.** | **PhOH[Gew.-%]** | **TPP-P[GeW.-%]** | **PhOH(g)** | **TPP-P*2PhOH(g)** | **T**_{**melt**} **[°C]** |
| 1 | 100 | 0 | 10,00 | 0,00 | 42 |
| 2 | 85 | 15 | 7,86 | 2,14 | 39 |
| 3 | 80 | 20 | 7,14 | 2,86 | 34 |
| 4 | 75 | 25 | 6,43 | 3,57 | 28 |
| 4a | 72 | 28 | 6,00 | 4,00 | ≤23 |
| 5 | 70 | 30 | 5,71 | 4,29 | ≤15 |
| 6 | 60 | 40 | 4,29 | 5,71 | ≤15 |
| 7 | 55 | 45 | 3,57 | 6,43 | ≤18 |
| 8 | 50 | 50 | 2,86 | 7,14 | 52 |
| 9 | 40 | 60 | 1,43 | 8,57 | 90 |
| 10 | 30 | 70 | 0,00 | 10,00 | 143 |

### Verwendungsbeispiele

### Herstellung von Schmelzpolycarbonat mit einer flüssigen TPP-P*2PhOH/PhOH-Katalysatorlösung

### Beispiel 1

In einem 500 ml Dreihalskolben mit Rührer, Innenthermometer und Vigreuxkolonne (30 cm, verspiegelt) mit Brücke wurden 114,15 g (0,500 mol) Bisphenol A und 113,54 g (0,530 mol) Diphenylcarbonat eingewogen. Die Apparatur wurde durch Anlegen von Vakuum und Spülen mit Stickstoff (3 mal) vom Luftsauerstoff befreit und das Gemisch auf 150° C aufgeheizt. Dann wurden 4x 10⁻³ mol-% Tetraphenylphosphoniumphenolat. bezogen auf Bisphenol A. in Form der flüssigen Mischung Nr. 6, zugegeben und das entstehende Phenol bei 100 mbar abdestilliert. Gleichzeitig wurde die Temperatur bis auf 250°C gesteigert. Nun wurde das Vakuum stufenweise bis auf 1 mbar verbessert und die Temperatur auf 260°C erhöht. Anschließend wurde die Temperatur auf 300°C erhöht und bei 0,1 mbar 1,5 Stunden gerührt. Man erhielt ein farbhelles lösungsmittelfreies Polycarbonat mit einer relativen Lösungsviskosität von 1,260 (Dichlormethan, 25°C, 5 g/l). Der Gehalt an Verzweiger der Formel (Ia) im hergestellten Polycarbonat betrug 25 ppm. Der phenolische OH-Wert des Polycarbonats betrug 70 ppm.

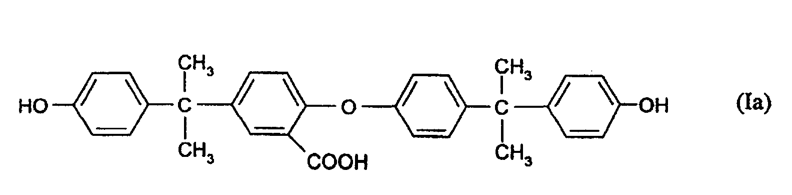

### Beispiel 2

Wie Beispiel 1, nur mit der Katalysatorlösung Nr. 5. Man erhielt ein farbhelles, lösungsmittelfreies Polycarbonat mit einer relativen Lösungsviskosität von 1,275 (Dichlormethan, 25°C, 5 g/l). Der Gehalt an Verzweiger der Formel (Ia) im hergestellten Polycarbonat betrug 18 ppm. Der phenolische OH-Wert des Polycarbonats betrug 55 ppm.

## Patentansprüche

1. Bei Raumtemperatur flüssige Katalysatorformulierung bestehend aus 28 bis 45 Gew.-% Tetraphenylphosphoniumphenolat und 72 bis 55 Gew.-% Phenol.

2. Verwendung der Katalysatorformulierung gemäß Anspruch 1 als Umesterungskatalysator.

3. Verwendung gemäß Anspruch 2, **dadurch gekennzeichnet, daß** die Katalysatorformulierung in Mengen von 0,01 bis 100000 ppm eingesetzt wird.

4. Verfahren zur Herstellung von thermoplastischen lösungsmittelfreien Polycarbonaten ausgehend von aromatischen Diphenolen, Kohlensäurediarylestem und gegebenenfalls Verzweigem und/oder Monophenolen, **dadurch gekennzeichnet, daß** als Umesterungskatalysator die Katalysatorformulierung gemäß Anspruch 1 in Mengen von 10⁻¹ bis 10⁻⁸ mol, bezogen auf 1 mol Diphenol, eingesetzt wird.

## Claims

1. Catalyst formulation which is liquid at room temperature, consisting of 28 to 45 wt.% tetraphenylphosphonium phenolate and 72 to 55 wt.% phenol.

2. Use of the catalyst formulation according to claim 1 as a transesterification catalyst.

3. Use according to claim 2, **characterized in that** the catalyst formulation is employed in amounts of 0.01 to 100,000 ppm.

4. Process for the preparation of thermoplastic solvent-free polycarbonates starting from aromatic diphenols, carbonic acid diaryl esters and optionally branching agents and/or monophenols, **characterized in that** the catalyst formulation according to claim 1 is employed as the transesterification catalyst in amounts of 10⁻¹ to 10⁻⁸ mol per mol of diphenol.

## Revendications

1. Formulation liquide à température ambiante de catalyseur, consistant en 28 à 45% en poids de phénolate de tétraphénylphosphonium et en 72 à 55% en poids de phénol.

2. Utilisation de la formulation de catalyseur suivant la revendication 1, comme catalyseur de transestérification.

3. Utilisation selon la revendication 2, **caractérisée en ce que** l'on met en oeuvre la formulation de catalyseur en des quantités allant de 0,01 à 100 000 ppm.

4. Procédé de préparation de polycarbonates thermoplastiques exempts de solvant, en partant de diphénols aromatiques, d'esters diaryliques d'acide carbonique et le cas échéant, d'agents de réticulation et/ou de monophénols, **caractérisé en ce que** comme catalyseur de transestérification, on met en oeuvre la formulation de catalyseur suivant la revendication 1, en des quantités allant de 10⁻¹ à 10⁻⁸ mole, sur base de 1 mole de diphénol.
